# EUROPEAN PATENT SPECIFICATION

(11) **EP 4 181 769 B1**
(45) Date of publication and mention of the grant of the patent: **03.09.2025**
(21) Application number: 21742063.7
(22) Date of filing: 30.06.2021
(51) Int. Cl.: A61B 5/00, A61B 5/25, A61B 5/30, A61B 5/304, A61B 5/305

(54) **APPARATUS FOR BIOPOTENTIAL MEASUREMENT**
VORRICHTUNG ZUR MESSUNG DES BIOPOTENTIALS
APPAREIL DE MESURE DE BIO-POTENTIEL

(30) Priority: 16.07.2020 US 202063052678 P
(43) Date of publication of application: 24.05.2023
(73) Proprietor: NEXTMIND SAS, 75009 Paris (FR)
(72) Inventor: JOLLET, Adrien, 75009 Paris (FR); PLOYART, Guillaume, 75009 Paris (FR)
(74) Representative: Kramer, Dani
(86) International application number: PCT/EP2021/068020
(87) International publication number: WO 2022/012935

(56) References cited:
- EP-A1- 0 835 635
- WO-A2-2014/205356
- US-A- 5 697 369
- US-A1- 2016 228 063

## Description

### Cross-Reference to Related Applications

This application claims the benefit of priority from U.S. Provisional Patent Application Serial Number 63/052,678, entitled "APPARATUS FOR BIOPOTENTIAL MEASUREMENT" and filed July 16, 2020.

### Technical field

Embodiments of the present disclosure relate to apparatus used in the acquisition of diffuse electrical signals. In particular, the apparatus is used in dynamic analog referencing and active user grounding for biopotential measurement in an electroencephalogram (EEG) acquisition headset.

EP 0835635 A1 describes a multi-lead ECG monitor that determines whether a reference electrode (LA) has been disconnected and, if so, switches to another reference electrode, which is selected in accordance with a predetermined priority scheme, to continue ECG monitoring. Disconnection of the reference electrode can be determined by observing that no ECG signal is being received from any of the electrodes.

### State of the art

Surface electroencephalography makes it possible to measure the variations of diffuse electric potentials on the surface of the skull of a subject. These variations of electrical potentials are commonly referred to as electroencephalogram signals or EEG signals.

EEG devices in the medical or related research fields generally include a close-fitting acquisition headset with attachment locations for receiving individual sensors/electrodes. Electronic circuits are then connected to the electrodes via cables and to the housing of an acquisition chain (i.e. an assembly of connected components used in acquiring the EEG signals).

Every time EEG acquisition headsets are worn the placement of the electrodes will vary. In turn, the relative electrical properties of the respective electrodes may vary. For this reason, it is usual to provide an electrode whose signal is designated as a reference. By choosing a reference that is relatively unaffected by the activity being measured but likely to be exposed to the same external electrical conditions, the effects of those external conditions may be excluded or indeed cancelled out.

In certain situations and with certain classes of electrodes, the use of one or more reference electrodes may expose the measurements made at measurement electrodes to sources of systematic error.

It is therefore desirable to provide apparatus for EEG devices that address the above challenges.

### SUMMARY

The present disclosure relates to apparatus that provide a balance of excellent signal quality from measurement electrodes, reliable referencing and active user grounding.

According to a first aspect, the present disclosure relates to an apparatus for generating a reference signal in a biopotential measurement system, the apparatus including an additive signal mixer for receiving respective electrode signals from each of a plurality of electrodes, each of the electrodes being configured to sense electric potentials on the surface of a subject, wherein the additive signal mixer is arranged to apply a respective weight to each electrode signal, and to sum the weighted signals, and wherein the sum of the weighted signals is supplied as a reference signal.

The additive signal mixer may include a switching unit and/or an averaging unit. The apparatus may further include electronic filtering and amplification circuits including a filtering unit and/or an amplification unit.

According to a second aspect, the present disclosure relates to a biopotential measurement system comprising: a plurality of electrodes, each of the electrodes being configured to sense electric potentials on the surface of a subject; a signal mixer for receiving respective electrode signals from each of the plurality of electrodes, the signal mixer being arranged to apply a respective weight to each electrode signal, to sum the weighted signals, and to output the sum of the weighted signals as a reference signal; a differential amplification module for receiving both the reference signal and the electrode signals from at least a subset of the electrodes and outputting corresponding referenced signals proportional to the difference between the respective electrode signals against the reference signal; a digitization module for transforming the referenced signals received from the differential amplification module into digital signals; and a microcontroller for transmitting the digital signals to an external processing unit.

The biopotential measurement system may be any one of an EEG device, an ECG/EKG device, an EMG device and an electrooculography (EOG) device.

According to a third aspect, the present disclosure relates to a method for generating a reference signal in a biopotential measurement system, the method comprising: receiving respective electrode signals from each of a plurality of electrodes, each of the electrodes being configured to sense electric potentials on the surface of a subject, applying a respective weight to each electrode signal, and summing the weighted signals to generate a reference signal. The present invention is set out in the appended claims.

### BRIEF DESCRIPTION OF THE SEVERAL VIEWS OF THE DRAWINGS

To easily identify the discussion of any particular element or act, the most significant digit or digits in a reference number refer to the figure number in which that element is first introduced.
FIG. 1 illustrates a conventional electronic architecture for receiving and processing EEG signals;
FIG. 2 shows a schematic diagram of a conventional acquisition subsystem for capturing and digitizing EEG signals;
FIG. 3 shows a schematic diagram of an acquisition subsystem for capturing and digitizing EEG signals according to an embodiment of the present disclosure;
FIG. 4 shows a schematic arrangement of an active dry electrode according to the present disclosure; and
FIG. 5 shows a flow of functional blocks according to the present disclosure.

### DETAILED DESCRIPTION

The description that follows includes systems, methods, techniques, instruction sequences, and computing machine program products that embody illustrative embodiments of the disclosure. In the following description, for the purposes of explanation, numerous specific details are set forth in order to provide an understanding of various embodiments of the inventive subject matter. It will be evident, however, to those skilled in the art, that embodiments of the inventive subject matter may be practiced without these specific details. In general, well-known instruction instances, protocols, structures, and techniques are not necessarily shown in detail.

Surface electroencephalography makes it possible to measure the variations of diffuse electric potentials (i.e. EEG signals) on the surface of the skull of a subject.

Extracting a reliable EEG signal, given the very small amplitude of the electrical potential variations to be measured (i.e. of the order of a few microvolts), drives development of surface EEG equipment that improves conductivity between sensors and the scalp and reliability of the contact under circumstances that vary over time. One obstacle to that improved contact can be condition (i.e. length, thickness and style) of the hair of the subject, which can significantly affect the impedance experienced by the measured electrical potentials. Another obstacle can be the physiology and mobility of the subject. Worn over any prolonged period, the contacts can be degraded by perspiration and the subject may be tempted to adjust the headset positioning for comfort.

To address the issue of signal reliability, some surface EEG devices are equipped with gel electrodes, in which contact is made through a gel or conductive liquid, which easily seeps through the user's hair to reach the scalp. The electrode itself is generally made of metal. The gel makes it possible to reduce the electrical impedance and thus the interference with surrounding signals without requiring physical contact between the electrode and the scalp. This solution provides good conductivity at any point of the scalp. Technical assistance is however required to ensure appropriate electrode placement, which in turn is time-consuming (since the gel must be applied and the conductance checked individually for each electrode).

In certain cases, the electrode includes circuitry (in addition to the metal "sensor" itself). This circuitry may process the analog signal coming from the sensor to amplify the electrical potentials captured at the sensor before that signal is routed to other electronic components. Such electrodes are termed the "active" wet electrodes. Amplification, in this context, means that a component strongly drives the voltage on a line to the level coming from the sensor. After amplification, the signal is less susceptible to interference as it is conveyed along connector cables to the other components before conversion (from analog to digital). The greater the impedance (due to hair condition, for example), the weaker the signal (in terms of driving voltage on the line) so this amplification circuitry can be essential for proper functioning even with gel.

The use of gel limits the duration of use of the device to a few hours (since the contact is no longer assured as the gel dries). In many cases, the conductive liquid or gel leaves a residue in the hair after the use of the EEG system, this can be difficult to remove.

More recently, surface electroencephalographs equipped with so-called "active dry" electrodes have been introduced: the electrodes being termed "dry" because they require no gel or other conductive liquid. Active dry electrodes operate by capturing the variations of electrical potential signals on the surface of the scalp, and then amplifying those signals (in some cases also filtering the signals). The analog signals thus obtained are then converted into digital signals by means of one or more analog-to-digital converters controlled by a microcontroller. The microcontroller receives the data for analysis, storage and/or onward transmission to another device.

In active dry electrodes, the contact with the scalp is through solid conducting elements or "sensors" connected to an electronic circuit to overcome the increase in impedance (compared to impedance in the presence of gel). The active dry electrode facilitates signal capture comparable to that of a gel electrode but also allows filtering and/or amplification of the captured signals, and thus an improved signal-to-noise ratio.

Stable access to the scalp limits the reliability of the signal capture. The shape of the sensors is restricted by the need for contact that extends through the hair of the subject.

It is known to provide pin-style active dry sensors (in a conductive polymer material) that require the application of significant pressure to reach the user's scalp. Such sensors are, due to the application of pressure at the interface of scalp and sensor pin, very uncomfortable, especially for prolonged use.

Medical and research EEG systems often use acquisition headsets in the form of an unflattering cap, often of elastic or waterproof fabric, with attachment locations for receiving individual sensors/electrodes. The EEG device therefore comprises electrodes (in a headset arrangement together with connecting cables), circuitry for digitizing, amplifying and/or filtering the signals captured at the electrodes and a microcontroller for processing the filtered transmission to an external processing unit. The EEG device is thus typically formed of three distinct elements that an operator / exhibitor must assemble at each use. Again, the nature of the EEG device is such that technical assistance is desirable if not essential.

Furthermore, user acceptability of the EEG device (and its electrodes) places aesthetic constraints, as well as constraints in comfort and ease of use. In many cases, these constraints are an effective and significant barrier to the adoption of EEG technology. Examples of applications where comfort over prolonged use and the need for technical assistance prevent adoption include applications such as video games, training (e.g. for health and safety or flight simulation), sleep aids, etc.

ECG/EKG (electrocardiography) is similar in some ways to EEG. Here, too, an arrangement of electrodes (typically 10 electrodes) is located on the skin surface of a subject (here the target of monitoring is the heart of the subject). The signals sensed at the ECG electrodes are potential differences that arise from changes in polarization in cardiac muscles as the heart beats. Signals may be sensed between pairs of the physical electrodes, such pairs being referred to as leads. The term "lead" is also used to refer to a pairing between a physical electrode and a "virtual electrode". One example of a virtual electrode in ECG is Wilson's central terminal: the potential measured by three difference physical electrodes (attached to the right arm, the left arm, and the left foot, respectively) is averaged to give the potential for that virtual electrode. Another example of a virtual electrode is Goldberger's central terminal which also combines inputs from electrodes attached to the limbs of the subject. Such virtual electrodes may be used as a negative pole in certain leads.

As for EEG, the surface electrical signals emitted by the cardiac muscles measured by ECG devices are very small, often as small as several micro-volts, so that it becomes important to reject common-mode voltages which might otherwise swamp the desired signals. The signals measured by the electrodes of ECG devices are equally sensitive to environmental interference - in particular, mains power at 50-60Hz - also referred to as common mode interference, CMI. As the subject's body is electrically floating, electromagnetic signals can couple to the subject's body, the cables and the circuitry of the ECG device, causing the potentials to drift relative to a reference voltage in the ECG device, affecting the performance of all electrodes in common. To address CMI, ECG systems often include additional circuitry that drives a current that actively cancels the interference (for instance, by monitoring the changing common voltage and driving a reference electrode at a voltage that mirrors the changing voltage around a known reference voltage). This driven current is applied through a physical electrode located at the subject's right leg - the right leg being the part of the body which is furthest from the heart - and the circuitry is termed the "driven right leg circuit" or DRL as a result. Electrodes paired with the right leg electrode through this circuit are used as a neutral lead in ECG. Usually the known reference voltage is half the supply voltage.

Circuitry on the same principle as the DRL circuit is frequently deployed to reduce common-mode interference in other biopotential measurement systems, such as EEG, electrooculography (EOG) and electromyography (EMG) systems.

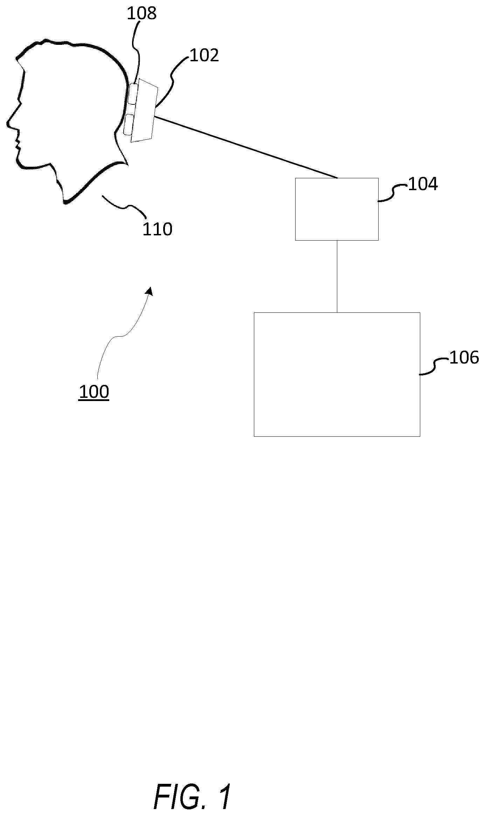

FIG. 1 illustrates an example of a conventional electronic architecture for the reception and processing of EEG signals by means of an EEG device 100.

To measure diffuse electric potentials on the surface of the skull of a subject 110, the EEG device 100 includes a portable device 102 (i.e. a cap or headpiece), analog-digital conversion (ADC) circuitry 104 and a microcontroller 106. The portable device 102 of FIG. 1 includes one or more active dry electrodes 108, typically between 1 and 128 electrodes, advantageously between 2 and 64, advantageously between 4 and 16.

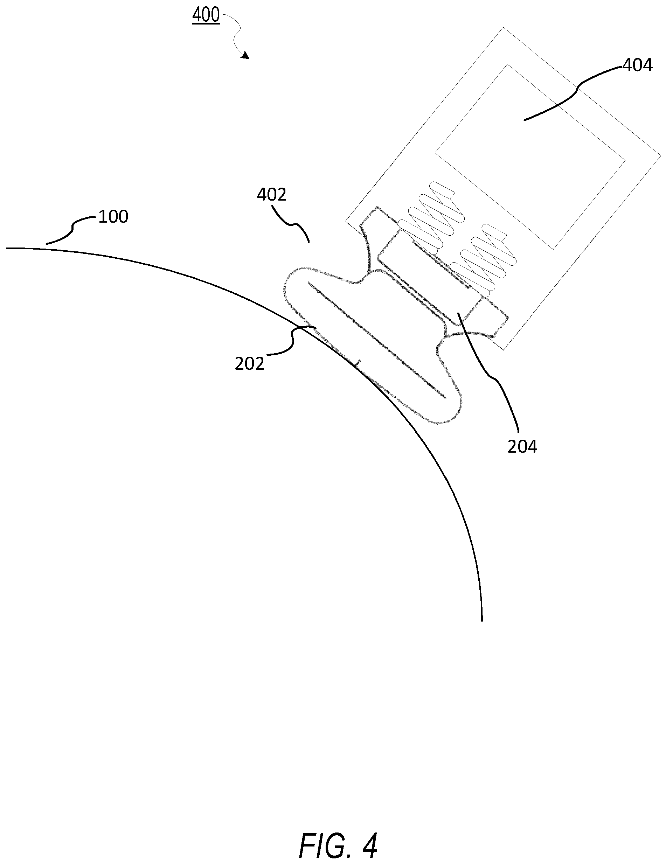

Each active electrode 108 comprises a sensor for detecting the electrical signals generated by the neuronal activity of the subject and an electronic filtering and amplifying circuit. These elements are discussed in more detail in relation to FIG. 4 below. The active electrodes 108 are shown in use in FIG. 1, where the sensor is in physical proximity with the subject's scalp.

Each ADC 104 is configured to convert the signals of a given number of active electrodes 108, for example between 1 and 128.

The converters 104 are controlled by the microcontroller 106 and communicate with it for example by the protocol SPI ("Serial Peripheral Interface"). The microcontroller 106 packages the received data for transmission to an external processing unit (not shown), for example a computer, a mobile phone, a virtual reality headset, an automotive or aeronautical computer system, for example a car computer or a computer system. airplane, for example by Bluetooth, Wi-Fi ("Wireless Fidelity") or Li-Fi ("Light Fidelity").

In certain embodiments, each active electrode 108 is powered by a battery (not shown in FIG. 1). The battery is conveniently provided in a housing of the portable device 102.

As discussed above, active dry electrodes make contact with the scalp of a subject through solid conducting elements or "sensors" connected to an electronic circuit. The sensors facilitate signal capture, while the corresponding electronic circuits allow amplification and/or filtering of the captured signals.

One or more of the active dry electrodes 108 are configured as "reference" electrodes (i.e. electrodes that assist in providing a reference level of electrical activity against which measurement may occur). The or each reference electrode is connected to the ADC 104. While any one of the electrodes at any location could be used as reference, it is good practice to select the reference with care because any activity in the reference electrode will reflected in the activity at other electrodes. The reference electrode or electrodes are preferably positioned in contact with the user's head in a region remote from that of the other active electrodes. In certain arrangements, the ADC circuitry 104 includes a differential amplification module and a digitization module. Each active (i.e. measurement) electrode 108 measures a respective electric potential value from which the potential measured by the reference electrode (Ei = Vi - Vref) is subtracted by the differential amplification module, and this difference value is digitized by means of the digitization module of the ADC 104 then transmitted by the microcontroller 106.

In certain cases (as illustrated in Fig. 1), the ADC 104 is provided in a single chip capable of performing both differential amplification and digitization. While not illustrated, the respective processes may be performed by separate components. In differential amplification, the reference potential is subtracted from the measuring electrode potential: in some cases, a gain may also be applied. The output of the differential amplification operation is a voltage value (i.e. a potential value referenced to the system's ground). Digitization, analog to digital conversion, which samples the differential measurement and translates this voltage value into a digital value.

In certain arrangements of electrodes for EEG devices, it may be convenient to provide more than one dedicated reference electrode. Conveniently, each dedicated reference electrode may take the same form as the measurement electrodes. The signal from any one or all of the reference electrodes may be used as a reference signal against which the potential difference at each of the measurement electrodes is measured.

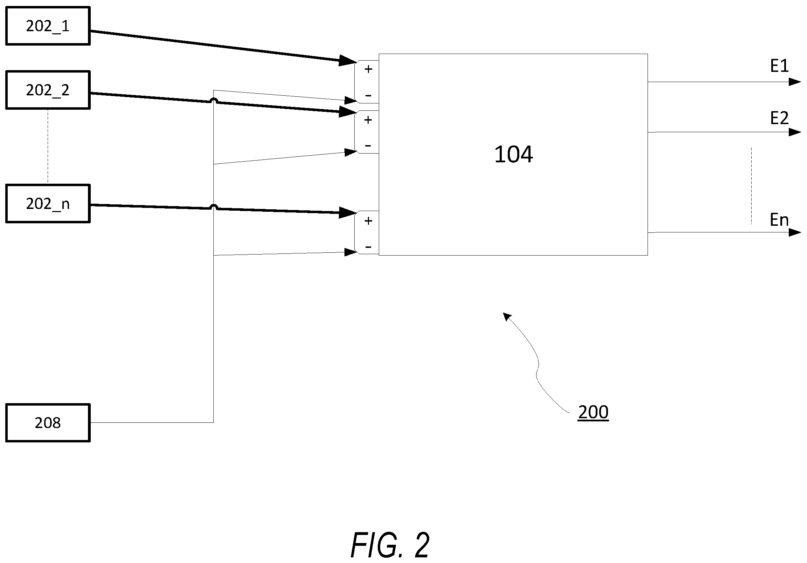

FIG. 2 shows a schematic diagram of a conventional acquisition subsystem for capturing and digitizing EEG signals in a conventional EEG system, such as the one illustrated in FIG. 1. For simplicity, the acquisition subsystem of FIG. 2 uses one specific electrode 208 as reference for all other measuring electrodes 202_1, 202_2, ... 200_n (202). This reference electrode 208 is connected to the negative input pins of all the channels of the ADC circuitry 104, where each positive input is connected to a respective measuring electrode 202.

In certain cases, it may be necessary to change which electrode should be considered as a reference against which all other electrodes potentials are measured. Furthermore, where none of the electrodes 202 can be relied upon as a permanent reference electrode it is common practice to take the average of activity at all electrodes as the reference value.

As EEG equipment measures a relative difference between physical quantities, (i.e. a potential difference between electric potentials), EEG measurement necessarily relies on some kind of hardware referencing before any meaningful signal can be successfully acquired. However, once signals have been acquired, EEG data analysis may include the application of a post-processing technique known as "re-referencing", which consists in computing differences between already-converted signals (i.e. signals after digital sampling in the ADC circuitry 104).

If the electrode configured as the physical reference electrode of the EEG acquisition subsystem is poorly chosen (either due to poor location or low contact quality), a common-mode interference will be visible on all measurements made relative to that reference. One way of handling this interference using re-referencing is to pick a measurement electrode channel that seems, after differential amplification and conversion (i.e. digitization), to carry the common-mode interference while not carrying any of the signal of interest and subtracting the signal from that channel from the signals on all other channels, leaving those other channels substantially interference-free. Re-referencing may thus be used to address common-mode interference in addition to (or in place of) DRL techniques discussed above.

There are however situations when no single channel can be found that carries the common-mode interference but does not also carry a valuable signal. In such cases, an alternative to that technique is to use instead the average value of all channels, which will have the effect of heightening every channel's difference from the others and once again cancelling out (i.e. rejecting) any common interference.

Alternatively, it might sometimes be useful to select a subset of electrodes of interest (e.g. electrodes empirically predicted to be most likely to capture signals of greatest interest) whose average value is subtracted from the values measured at all other measurement electrodes. This will effectively weight the signal portion specific to these electrodes' location while lowering global activity, once again reducing common mode interference.

For any kind of re-referencing strategy to be effective, signals have to be correctly converted in the first place. While this may be easily achievable under ideal conditions, both in electrode placement and in contact quality (which can be achieved using a conventional EEG cap with conductive gel applied between electrodes and scalp), it becomes far more challenging when dealing with dry electrodes or/and closely packed electrodes.

A poor contact quality of the reference electrode might lead to higher differential voltages to other electrodes, in the form of both a DC offset and potentially great fluctuations caused by an increased sensitivity to interfering environmental electric fields, most commonly originating in mains power lines and statically charged objects/persons in the user's surroundings.

Such differential voltages might lead to the permanent or intermittent saturation of differential amplifiers inputs or outputs (e.g. in a differential amplification module) if any differential gain is applied before analog to digital conversion (as would be typical with active dry electrodes). This will cause output digital signals acquired to be either constant or clipped, with inherent information loss in either case. Conventional re-referencing would thus prove unable to recover any of the valuable lost data, unlike a change in the actual electronic reference.

A further issue with re-referencing using a conventional physical reference electrode arises from the potentially negative impact upon resulting digital signals of calculating noise contributions in multiple conversions over a single value (i.e. that measured at the physical reference electrode).

To illustrate this noise calculation problem, consider a situation where the most relevant data resides in a specific differential setup (between an optimal reference electrode or electrodes average, and an optimal measure electrode), where the potential at the optimal measuring electrode is V1; the optimal reference potential is V0, and the potential of a fixed hardware reference is Vref. Re-referencing on a fixed reference system will use both the converted signals of V1-Vref and V0-Vref, to compute (whether in real-time or in offline analysis) V1-V0 by subtracting (V0-Vref) from (V1-Vref).

In an ideal system there would be no difference between the above calculation of the converted value of V1-V0 and the direct conversion to V1-V0: the resulting signal would be identical. In practical systems, there are differences due to the inherent degradation of data occurring in analog to digital conversions. The finite precision of real-world converters gives rise to a quantization noise, which can be expressed as a noise being superimposed over the original analog signals. This noise will be directly linked to the resolution of the converters used in the system. Other inherent converter defects include added thermal noise and imperfect linearity between input voltages and output codes (INL : integral non-linearity). The added noise is different for every acquired signal. Rather than cancelling out the added noise, re-referencing, means that the quantization noise components will add up. Quantization noise is usually considered close to a white noise, i.e. flat in its frequency contributions. This means that it cannot be simply filtered out because it will be present in any frequency band of interest.

While quantization noise is usually negligible with high resolution converters, it can become significant and lower the overall signal to noise ratio in a system where the resolution of conversions is a limiting factor.

Once again, this problem might be avoided when using active wet electrodes because of smaller swings in potential differences between electrodes, allowing for more differential gain to be applied before the converter stage without risking saturation of the amplifying electronics or the ADC inputs.

Dry electrode systems call for greater headroom, meaning less resolution will be available for the signals of interest themselves. For example, a 24bit high-resolution ADC might be used to convert differential voltages of several hundreds of millivolts, when EEG signals are usually a few hundreds of microvolts in amplitude at most, lowering the effective resolution by around 10bits.

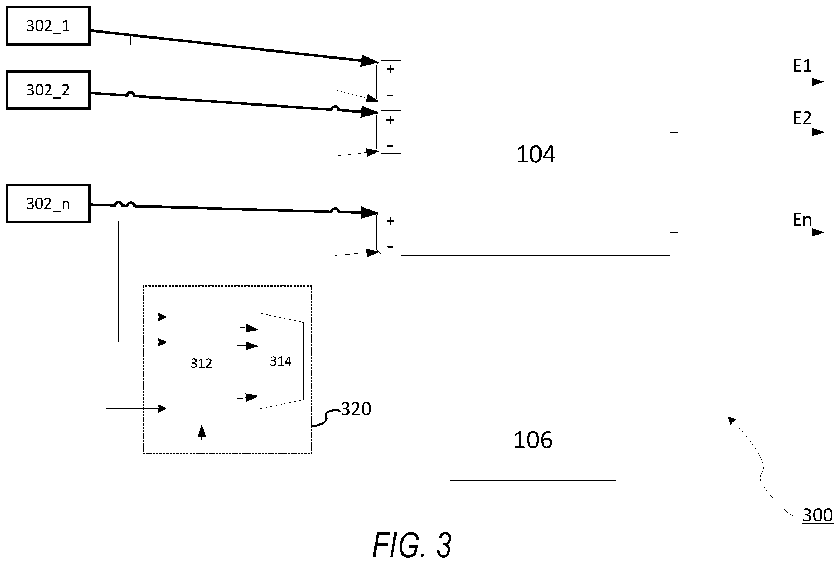

FIG. 3 shows a schematic diagram of an acquisition subsystem for capturing and digitizing EEG signals according to an embodiment of the present disclosure. The acquisition subsystem of FIG. 3 has no dedicated reference electrode. Here each of a plurality of measuring electrodes 302_1, 302_2, ... 302_n (302) is connected to a respective positive input pin of the channels of the ADC circuitry 104. Each of the n signals from the plurality of measuring electrodes 302 is also input into a respective one of the n input ports of an additive signal mixer 320.

The additive signal mixer 320 includes a switching unit 312 and an averaging unit 314. The switching unit 312 includes a plurality of controllable switches which selectively allow or bar the transmission of the n input signals to a corresponding output port of the switching unit 312. The output signal at each of the n output ports of the switching unit 312 is then input to the averaging unit 314.

The averaging unit 314 operates to perform an averaging function upon the non-zero output signals from the switching unit 312. The averaging unit 314 then outputs an averaged signal output that is applied to the negative input pins of all the channels of the ADC circuitry 104. Where the averaging function is a simple average, the averaged signal output is therefore a real-time average of the signals captured at a selected subset of the electrodes 302. The averaging function may combine the signals of the selected subset of electrodes in a number of different ways. The selected subset of electrodes may, for example, be a subset of electrodes placed far from the region of interest on the user's head or a number of electrodes placed at the opposite side of the region of interest to benefit from an inversion of the polarity of the electrical activity of interest.

The switching unit 312 and averaging unit 314 may be implemented in separate analog circuitry. Alternatively, they may be implemented in a single integrated circuit. The integrated circuit may also incorporate the ADC 104. Control of the switching unit 312 and averaging unit 314 may also be implemented in software or firmware executed in a microcontroller. Switching and averaging operations are however applied to analog signals before referencing (i.e. differential amplification) and consequently these operations are applied before analog to digital conversion.

In certain embodiments, the switching unit 312 is a digitally controlled n-channel switch and the averaging unit 314 is an averaging resistor network. The switching in the *n-*channel switch is thus controlled by a microcontroller unit (such as the microcontroller 106 in FIG. 1) whose function is to select in real time which electrode should be included in the subset of electrodes implementing the virtual reference electrode. The electric potentials captured by the selected subset of electrodes are hence combined (e.g. averaged out) to build the reference electrode signal. In certain embodiments, the averaging unit 314 includes a plurality of digitally controllable resistors, each resistor independently being able to take a range of resistance values, thereby allowing the output of a reference signal using a weighted average value from the selected subset of electrodes. Where the averaging unit 314 gives equal weight to each input signal, the output reference signal will be proportional to the arithmetic mean of those signals.

In the electrode arrangement of FIG. 3, then, there is no dedicated reference electrode. Instead, the captured signals from each of a hardware-selectable subset of the n electrodes of the EEG headset are used to implement a dynamically-defined "virtual" reference electrode, for instance by averaging the captured signals from each of the subset of electrodes.

By controlling, in real-time, the selection of electrodes from which a reference signal is to be constructed, the resulting reference signal may be advantageously used in re-referencing and DRL strategies for rejection of CMI.

Compared to conventional systems using one or more physical reference electrodes, re-referencing can benefit from the above-described hardware control of the reference signal by allowing for more accurate initial conversion of the electrode signals upon which the re-referencing is to be performed and by limiting exposure of the system to quantization noise contributions.

In the context of DRL strategies, a virtual reference signal generated as above may be used as a means of efficiently sensing variations in the potential difference between user and system. This potential difference can be used as the reference for a common design in Driven Right Leg, DRL, circuits.

The hardware-controlled reference signal may serve not only as an EEG reference potential for measurements but also for patient/user grounding.

As noted above DRL is a common technique used not only in EEG but in physiological biopotentials measurement systems (such as ECG, EMG, EOG). The DRL technique operates to actively reduce the average potential difference between user (e.g. the human subject of the biopotential measurement) and measurement instrumentation by forming a closed loop in which the user potential relative to system ground is measured and used to adjust the resistive path between them through the use of an electronic amplifier.

Historically, that path to ground was first made using a simple "ground" electrode directly connected to the system's ground. The drawback of this implementation was the inevitable potential difference across this electrode's contact resistance, leading to poor performances and thus large common-mode swings at the measurement electrodes locations.

Conventional DRL architectures include an added CMS electrode (for Common Mode Sense) dedicated to picking up the gap in potential across the contact resistance of the grounding electrode, and an amplifier whose role is to adjust the resistive path between the grounding electrode, now called DRL electrode, connected to its output, and its own power supply rails, those also being the supply rails for the measurement differential amplifiers.

The DRL architecture is thus unanimously recognized as the best way to prevent a measurement differential amplifier's inputs saturation by common-mode interferences pushing the measured potentials beyond the supply rails of the measurement device. It is seen as crucial in any system susceptible to large swings in user-system potential difference or that have design limitations in terms of either/both power supply voltages or poor electrode contact quality, such as battery powered or/and dry-electrode wearable devices.

Although DRL circuitry can be constructed to compensate for poor contact with the user's body (e.g. the subject's head, in EEG examples), the CMS electrode cannot be made similarly tolerant. Attempting to do so might result in a CMS electrode that is more sensitive to interference than the measurement electrodes, making the whole strategy pointless.

Some DRL architectures try to counter this problem by using a fixed, average potential of all measuring electrodes. Although this somewhat addresses the problem of a single defective electrode contact, it does so by assuming good overall contact quality of the other electrodes: this method still proves inefficient in systems where there are not enough electrodes having good overall contact quality to counteract the level of interference picked up by the faulty ones (i.e. those with poor overall contact quality).

This is where an ability to change which electrode potential serves as the CMS potential comes in handy, especially in systems where good ohmic contact of any particular electrode cannot be expected at all times.

As there are no particular requirements on the CMS potential (whether it's coming from a single or the average of multiple electrodes potentials) that do not also apply to the reference potential, both can be the same. Indeed, this is the case with the widely used Biosemi ActiveTwo EEG-system (Biosemi BV, Amsterdam, Netherlands), among others. Consequently, the dynamic hardware control of the reference signal of the present disclosure may be employed for both referencing and/or common-mode sensing, with no apparent interoperability issues.

FIG. 4 illustrates an arrangement of functional elements of an active dry electrode 400 including a sensor 402 and an electronic circuit 404. The active dry electrode may be used as one of the one or more active dry electrodes 108 in FIG. 1 or 302 in FIG. 3.

In certain embodiments, each electronic circuit 404 comprises a first-order high-pass analog filter, an amplifier and a first-order low-pass analog filter. The filters make it possible to suppress signals that are detected by the frequency components that are useless for the intended application.

The active dry electrode 400 is shown in contact with the scalp of the subject 100. This may be at a localized region of the subject's head, for example, the occipital region at the rear of the skull.

The amplification at each electronic circuit 404 makes it possible to adapt the amplitude of the signals to the characteristics of the analog-digital converter (ADC) (104 in FIGs. 1, 2 & 3), and to obtain a maximum resolution during the conversion.

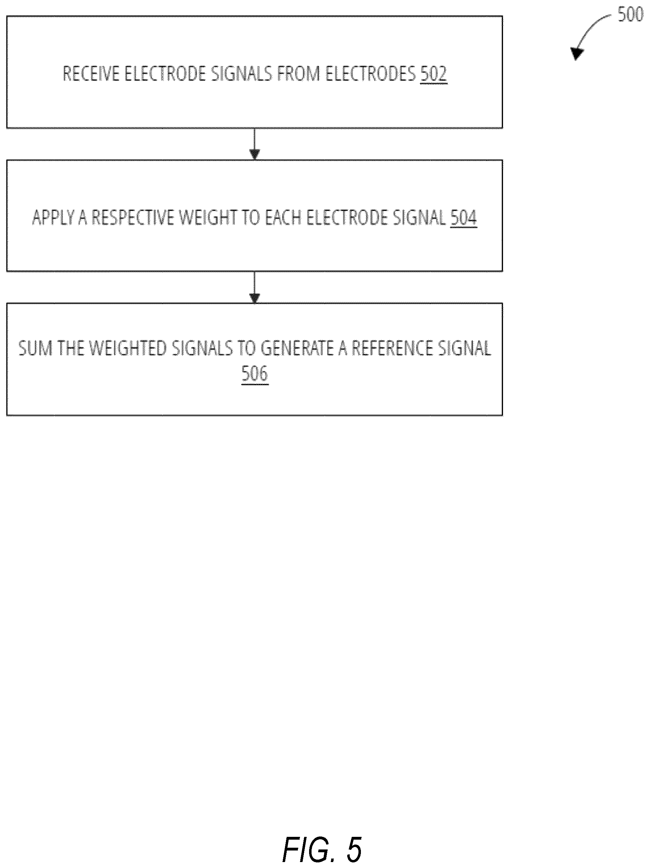

FIG. 5 shows a flow of functional blocks according to the present disclosure. In block 502, FIG. 5 shows a flow of functional blocks according to the present disclosure. In block 502, an additive signal mixer receives respective electrode signals from each of a plurality of electrodes. Each of the electrodes is configured to sense electric potentials on the surface of a subject. The electrodes may be active dry electrodes in an EEG acquisition headset, for example.

At block 504, a respective weight is applied to each electrode signal. The weight may include a zero weight, a zero weighted electrode signal being effectively switched off. The additive signal mixer may include a switching unit, zero-weighting of a given electrode may be effected by opening (i.e. switching OFF) a corresponding switch in the switching unit.

At block 506, the weighted signals are summed by the additive mixer to generate a reference signal. The reference signal is therefore a weighted average value from the plurality of electrodes.

Although described through a number of detailed exemplary embodiments, the portable devices for the acquisition of electroencephalographic signals according to the present disclosure comprise various variants, modifications and improvements which will be obvious to those skilled in the art, it being understood that these various variants, modifications and improvements fall within the scope of the subject of the present disclosure, as defined by the following claims.

Throughout the description and claims of this specification, the words "comprise" and "contain" and variations of the words, for example "comprising" and "comprises", mean "including but not limited to", and are not intended to (and does not) exclude other components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers or characteristics described in conjunction with a particular aspect, embodiment or example of the invention are to be understood to be applicable to any other aspect, embodiment or example described herein unless incompatible therewith.

It will also be appreciated that, throughout the description and claims of this specification, language in the general form of "X for Y" (where Y is some action, activity or step and X is some means for carrying out that action, activity or step) encompasses means X adapted or arranged specifically, but not exclusively, to do Y. The present invention is set out in the claims that follow.

## Claims

1. An apparatus for generating a reference signal in a biopotential measurement system with no physical reference electrode,
the apparatus including an additive signal mixer (320) for receiving respective electrode signals from each of a plurality of electrodes (302) of the biopoetential measurement system, each of the electrodes being configured to sense electric potentials on the surface of a subject,
wherein the additive signal mixer (320) is arranged to apply a respective weight to each electrode signal, and to sum the weighted signals, and
wherein the sum of the weighted signals is supplied as a virtual reference signal to the biopotential measurement system.

2. The apparatus of claim 1, wherein the additive signal mixer (320) includes a switching unit (312), the switching unit (312) being configured to receive the respective electrode signals from each of the plurality of electrodes (302) and to output a first subset of the electrode signals.

3. The apparatus of claim 2, wherein the additive signal mixer (320) further includes an averaging unit (314), the averaging unit (314) being configured to receive the first subset of the electrode signals, to apply the respective weight to each of the first subset of the electrode signals, and to sum the weighted first subset of the electrode signals, the sum of the weighted first subset of the electrode signals being supplied as the reference signal.

4. The apparatus of any one of the preceding claims, wherein the weight applied to a second subset of the plurality of electrode signals is zero.

5. The apparatus of any one of the preceding claims, wherein the respective weights applied to each electrode signal are controlled by a controller, preferably wherein the respective weights are controlled in real-time.

6. The apparatus of any one of the preceding claims, wherein the reference signal is supplied to a negative port of an analog to digital converter, ADC, (104), the ADC (104) receiving at least one of the respective electrode signals at a positive port corresponding to the negative port.

7. The apparatus of any one of the preceding claims further including a filtering unit for suppressing frequency components of the electrode signals outside a predetermined range of frequencies, the additive signal mixer receiving respective electrode signals from each of a plurality of electrodes via the filtering unit.

8. The apparatus of claim 7, wherein the filtering unit includes at least one of a low-pass filter, a high-pass filter and a band-pass filter.

9. The apparatus of claim 7 or claim 8 further including an amplification unit, the amplification unit comprising at least one amplifier for amplifying the electrode signals, the additive signal mixer receiving respective electrode signals from each of a plurality of electrodes via the amplification unit.

10. The apparatus of any one of the preceding claims, wherein the reference signal is used as a base reference for a re-referencing procedure.

11. The apparatus of any one of the preceding claims, wherein the reference signal is used as a reference voltage in driven right leg common-mode interference rejection.

12. A biopotential measurement system with no physical reference electrode comprising:
a plurality of electrodes, each of the electrodes being configured to sense electric potentials on the surface of a subject;
the apparatus of any preceding claim;
a differential amplification module for receiving both the reference signal and the electrode signals from at least a subset of the electrodes and outputting corresponding referenced signals proportional to the difference between the respective electrode signals against the reference signal;
a digitization module for transforming the referenced signals received from the differential amplification module into digital signals; and
a microcontroller for transmitting the digital signals to an external processing unit.

13. The biopotential measurement system of claim 12, wherein the biopotential measurement system is an electroencephalogram, EEG, system.

14. A method for generating a reference signal in a biopotential measurement system with no physical reference electrode, the method comprising:
receiving (502) respective electrode signals from each of a plurality of electrodes (302) of the biopotential measurement system, each of the electrodes being configured to sense electric potentials on the surface of a subject,
applying (504) a respective weight to each electrode signal, and
summing (506) the weighted signals to generate a virtual reference signal for being supplied to the biopotential measurement system.

15. The method of claim 14, wherein applying (504) the respective weight includes applying a non-zero weight to a first subset of the electrode signals.

## Patentansprüche

1. Vorrichtung zur Erzeugung eines Referenzsignals in einem Biopotentialmesssystem ohne physische Referenzelektrode,
wobei die Vorrichtung einen Additivsignalmischer (320) zum Empfangen von jeweiligen Elektrodensignalen von jeder von einer Vielzahl von Elektroden (302) des Biopotentialmesssystems einschließt, wobei jede der Elektroden dazu konfiguriert ist, elektrische Potentiale auf der Oberfläche eines Probanden zu erfassen,
wobei der Additivsignalmischer (320) dazu eingerichtet ist, eine jeweilige Gewichtung auf jedes Elektrodensignal anzuwenden und die gewichteten Signale zu summieren, und
wobei die Summe der gewichteten Signale als ein virtuelles Referenzsignal zu dem Biopotentialmesssystem zugeführt wird.

2. Vorrichtung nach Anspruch 1, wobei der Additivsignalmischer (320) eine Schalteinheit (312) einschließt, wobei die Schalteinheit (312) dazu konfiguriert ist, die jeweiligen Elektrodensignale von jeder von der Vielzahl von Elektroden (302) zu empfangen und einen ersten Teilsatz der Elektrodensignale auszugeben.

3. Vorrichtung nach Anspruch 2, wobei der Additivsignalmischer (320) ferner eine Mittelungseinheit (314) einschließt, wobei die Mittelungseinheit (314) dazu konfiguriert ist, den ersten Teilsatz der Elektrodensignale zu empfangen, die jeweilige Gewichtung auf jedes von dem ersten Teilsatz der Elektrodensignale anzuwenden und den gewichteten ersten Teilsatz der Elektrodensignale zu summieren, wobei die Summe des gewichteten ersten Teilsatzes der Elektrodensignale als das Referenzsignal zugeführt wird.

4. Vorrichtung nach einem der vorhergehenden Ansprüche, wobei die Gewichtung, die auf einen zweiten Teilsatz der Vielzahl von Elektrodensignalen angewendet wird, null ist.

5. Vorrichtung nach einem der vorhergehenden Ansprüche, wobei die jeweiligen Gewichtungen, die auf jedes Elektrodensignal angewendet werden, von einem Controller gesteuert werden, vorzugsweise wobei die jeweiligen Gewichtungen in Echtzeit gesteuert werden.

6. Vorrichtung nach einem der vorhergehenden Ansprüche, wobei das Referenzsignal zu einem negativen Anschluss eines Analog-Digital-Umsetzer, ADU (104), zugeführt wird, wobei der ADU (104) mindestens eines der jeweiligen Elektrodensignale an einem positiven Anschluss empfängt, der dem negativen Anschluss entspricht.

7. Vorrichtung nach einem der vorhergehenden Ansprüche, ferner einschließend eine Filtereinheit zum Unterdrücken von Frequenzkomponenten der Elektrodensignale außerhalb eines vorbestimmten Bereichs von Frequenzen, wobei der Additivsignalmischer jeweilige Elektrodensignale von jeder von einer Vielzahl von Elektroden mittels der Filtereinheit empfängt.

8. Vorrichtung nach Anspruch 7, wobei die Filtereinheit mindestens einen von einem Tiefpassfilter, einem Hochpassfilter und einem Bandpassfilter einschließt.

9. Vorrichtung nach Anspruch 7 oder Anspruch 8, ferner einschließend eine Verstärkungseinheit, wobei die Verstärkungseinheit mindestens einen Verstärker zum Verstärken der Elektrodensignale umfasst, wobei der Additivsignalmischer jeweilige Elektrodensignale von jeder von einer Vielzahl von Elektroden mittels der Verstärkungseinheit empfängt.

10. Vorrichtung nach einem der vorhergehenden Ansprüche, wobei das Referenzsignal als eine Basisreferenz für einen Neureferenzierungsvorgang verwendet wird.

11. Vorrichtung nach einem der vorhergehenden Ansprüche, wobei das Referenzsignal als eine Referenzspannung in einer Driven-Right-Leg-Gleichtakt-Interferenzunterdrückung verwendet wird.

12. Biopotentialmesssystem ohne physische Referenzelektrode, umfassend:
eine Vielzahl von Elektroden, wobei jede der Elektroden dazu konfiguriert ist, elektrische Potentiale auf der Oberfläche eines Probanden zu erfassen;
die Vorrichtung nach einem vorhergehenden Anspruch;
ein Differentialverstärkungsmodul zum Empfangen sowohl des Referenzsignals als auch der Elektrodensignale von mindestens einem Teilsatz der Elektroden und Ausgeben von entsprechenden referenzierten Signalen proportional zu der Differenz zwischen den jeweiligen Elektrodensignalen gegen das Referenzsignal;
ein Digitalisierungsmodul zum Umwandeln der referenzierten Signale, die von dem Differentialverstärkungsmodul empfangen werden, in digitale Signale; und
einen Mikrocontroller zum Übertragen der digitalen Signale an eine externe Verarbeitungseinheit.

13. Biopotentialmesssystem nach Anspruch 12, wobei das Biopotentialmesssystem ein Elektroenzephalographiesystem, EEG-System, ist.

14. Verfahren zur Erzeugung eines Referenzsignals in einem Biopotentialmesssystem ohne physische Referenzelektrode, wobei das Verfahren umfasst:
Empfangen (502) von jeweiligen Elektrodensignalen von jeder von einer Vielzahl von Elektroden (302) des Biopotentialmesssystems, wobei jede der Elektroden dazu konfiguriert ist, elektrische Potentiale auf der Oberfläche eines Probanden zu erfassen,
Anwenden (504) einer jeweiligen Gewichtung auf jedes Elektrodensignal, und
Summieren (506) der gewichteten Signale, um ein virtuelles Referenzsignal zu erzeugen, das zu dem Biopotentialmesssystem zuzuführen ist.

15. Verfahren nach Anspruch 14, wobei das Anwenden (504) der jeweiligen Gewichtung ein Anwenden einer Nicht-Null-Gewichtung auf einen ersten Teilsatz der Elektrodensignale einschließt.

## Revendications

1. Appareil destiné à générer un signal de référence dans un système de mesure de biopotentiel sans électrode de référence physique,
l'appareil incluant un mélangeur de signaux additifs (320) destiné à recevoir des signaux d'électrode respectifs de chacune d'une pluralité d'électrodes (302) du système de mesure de biopotentiel, chacune des électrodes étant configurée pour détecter des potentiels électriques à la surface d'un sujet,
dans lequel le mélangeur de signaux additifs (320) est agencé pour appliquer un poids respectif à chaque signal d'électrode, et pour additionner les signaux pondérés, et
dans lequel la somme des signaux pondérés est fournie comme un signal de référence virtuel au système de mesure de biopotentiel.

2. Appareil selon la revendication 1, dans lequel le mélangeur de signaux additifs (320) inclut une unité de commutation (312), l'unité de commutation (312) étant configurée pour recevoir les signaux d'électrode respectifs de chacune de la pluralité d'électrodes (302) et pour émettre en sortie un premier sous-ensemble des signaux d'électrode.

3. Appareil selon la revendication 2, dans lequel le mélangeur de signaux additifs (320) inclut en outre une unité de calcul de moyenne (314), l'unité de calcul de moyenne (314) étant configurée pour recevoir le premier sous-ensemble des signaux d'électrode, pour appliquer le poids respectif à chacun du premier sous-ensemble des signaux d'électrode, et pour additionner le premier sous-ensemble pondéré des signaux d'électrode, la somme du premier sous-ensemble pondéré des signaux d'électrode étant fournie comme le signal de référence.

4. Appareil selon l'une quelconque des revendications précédentes, dans lequel le poids appliqué à un deuxième sous-ensemble de la pluralité de signaux d'électrode est nul.

5. Appareil selon l'une quelconque des revendications précédentes, dans lequel les poids respectifs appliqués à chaque signal d'électrode sont commandés par un dispositif de commande, de préférence dans lequel les poids respectifs sont commandés en temps réel.

6. Appareil selon l'une quelconque des revendications précédentes, dans lequel le signal de référence est fourni à un port négatif d'un convertisseur analogique-numérique, CAN, (104), le CAN (104) recevant au moins l'un des signaux d'électrode respectifs au niveau d'un port positif correspondant au port négatif.

7. Appareil selon l'une quelconque des revendications précédentes incluant en outre une unité de filtrage pour supprimer des composantes de fréquence des signaux d'électrode en dehors d'une plage de fréquences prédéterminée, le mélangeur de signaux additifs recevant des signaux d'électrode respectifs provenant de chacune d'une pluralité d'électrodes via l'unité de filtrage.

8. Appareil selon la revendication 7, dans lequel l'unité de filtrage inclut au moins l'un d'un filtre passe-bas, d'un filtre passe-haut et d'un filtre passe-bande.

9. Appareil selon la revendication 7 ou la revendication 8 incluant en outre une unité d'amplification, l'unité d'amplification comprenant au moins un amplificateur destiné à amplifier les signaux d'électrode, le mélangeur de signaux additifs recevant des signaux d'électrode respectifs de chacune d'une pluralité d'électrodes via l'unité d'amplification.

10. Appareil selon l'une quelconque des revendications précédentes, dans lequel le signal de référence est utilisé comme une référence de base pour une procédure de réréférencement.

11. Appareil selon l'une quelconque des revendications précédentes, dans lequel le signal de référence est utilisé comme une tension de référence dans le rejet d'interférence en mode commun de la jambe droite entraînée.

12. Système de mesure de biopotentiel sans électrode de référence physique comprenant :
une pluralité d'électrodes, chacune des électrodes étant configurée pour détecter des potentiels électriques sur la surface d'un sujet ;
l'appareil selon l'une quelconque des revendications précédentes ;
un module d'amplification différentielle destiné à recevoir à la fois le signal de référence et les signaux d'électrode d'au moins un sous-ensemble des électrodes et à émettre en sortie des signaux référencés correspondants proportionnels à la différence entre les signaux d'électrode respectifs par rapport au signal de référence ;
un module de numérisation destiné à transformer les signaux référencés reçus du module d'amplification différentielle en signaux numériques ; et
un dispositif de microcommande destiné à transmettre les signaux numériques à une unité de traitement externe.

13. Système de mesure de biopotentiel selon la revendication 12, dans lequel le système de mesure de biopotentiel est un système d'électroencéphalogramme, EEG.

14. Procédé destiné à générer un signal de référence dans un système de mesure de biopotentiel sans électrode de référence physique, le procédé comprenant :
la réception (502) de signaux d'électrode respectifs de chacune d'une pluralité d'électrodes (302) du système de mesure de biopotentiel, chacune des électrodes étant configurée pour détecter des potentiels électriques à la surface d'un sujet,
l'application (504) d'un poids respectif à chaque signal d'électrode, et
la somme (506) des signaux pondérés pour générer un signal de référence virtuel destiné à être fourni au système de mesure de biopotentiel.

15. Procédé selon la revendication 14, dans lequel l'application (504) du poids respectif inclut l'application d'un poids non nul à un premier sous-ensemble des signaux d'électrode.
